# EUROPEAN PATENT APPLICATION

(11) **EP 4 813 997 A1**
(43) Date of publication of application: **30.09.2026**
(21) Application number: 25167120.2
(22) Date of filing: 28.03.2025
(51) Int. Cl.: C07H 1/00, C07H 19/173

(54) **6C-MODIFIED PURINE NUCLEOSIDES AND METHODS OF SYNTHESIS THEREOF**

(71) Applicant: Universität zu Köln, 50923 Köln (DE)
(72) Inventor: Kath-Schorr, Stephanie, 45527 Hattingen (DE); Behn, Tobias, 50823 Köln (DE)
(74) Representative: CH Kilger Anwaltspartnerschaft mbB

(57) **Abstract**

The invention relates to a method of synthesizing of 6C modified guanosine analogues, wherein a) the guanosine analogue comprises 2'deoxy-ribose or ribose, b) the guanosine analogue is protected at the 5' position by DMT group (dimethoxytrityl), c) the guanosine analogue is phosphorylated at 3' position with (2-cyanoethoxy)(diisopropylamino)phosphaneyl-group, d) the guanosine analogue is substituted at the amino group of 2C position with 2-methyl-1-oxopropyl group, and e) the 6C modification of the guanosine analogue is selected from the group of a. a heterocycle (C1); b. a 5-membered heterocycle comprising W, X, Y and Z, wherein W, X, Y and Z can be C, N, O and S (D1); c. an oxadiazole (D2); d. a BOC-imidazole (B1) and e. BOC-pyrazole (B2).

## Description

### BACKGROUND

In addition to encoding genetic information, nucleosides play a central role in cell metabolism. The binding motifs of these nucleosides are associated with a broad array of targets of therapeutic importance in biological systems. Thus, nucleoside analogues can be used for example as inhibitors of these processes, for example DNA biosynthesis, a process that is essential for cell growth and viral replication. They can be used in biochemical molecular applications such as DNA or RNA synthesis or sequencing. They can be used also for synthesizing previously unknown Deoxyribozymes, also called DNA enzymes, DNAzymes, or catalytic DNA, which are DNA oligonucleotides that are capable of performing a specific chemical reaction, often but not always catalytic. This is similar to the action of other biological enzymes, such as proteins or ribozymes (enzymes composed of RNA).

Naturally occurring nucleoside analogs demonstrate selective activities such as protein synthesis inhibition (puromycin), glycosyl transferase inhibition (tunicamycin) and methyltransferase inhibition (sinefungin). Similarly, non-naturally occurring nucleoside analogues are known to be therapeutically useful, for example as antipsychotics, cardiotonics, diuretics, analgesic, antiinflammatory agents, anticonvulsants, antihypertensives, antibiotics, antivirals, and anticancer agents. Many of these nucleoside analogues are either on the market or in advanced clinical stages.

Generally, there are two ways to prepare a nucleoside analogue. The first way follows a linear synthetic sequence in which the target nucleoside is prepared from an appropriate nucleoside. In this approach, usually there is less concern about stereoselective chemistry as most if not all of the stereocenters are set. However, the synthesis can be lengthy if extensive modification of the sugar is required.

An alternative approach toward the synthesis of novel nucleosides utilizes a convergent synthesis where a sugar portion is separately modified and later coupled with an appropriate silylated base.

Another possible way to do an S_{N}2 type coupling is enzymatic glycosylation in which the sugar- 1-α-O-phosphate is coupled with purine base using either isolated enzymes or whole cells. The phosphate intermediate can be generated enzymatically from another nucleoside containing the desired sugar. This coupled reaction is called transglycosylation. This conversion is highly stereospecific. Unfortunately, natural enzymes only work with a limited number of modified sugars. For custom sugars, existing enzymes from a range of microorganisms need to be screened for activity or through extensive research there is a possibility that a mutated enzyme can be selected and produced though genetic engineering. This approach does not allow for heavily modified nucleosides as this approach depends on molecules which will be accepted by the enzymes.

An alternative method to couple a sugar with a purine base is through the use of Mitsunobu chemistry. This approach uses a condensing reagent such as N,N-dicyclohexylcarbodiiomide (DCC) and triphenylphosphine. Although this reaction accepts a wide variety of substrates, yields are typically lower and there is no stereoselectivity. Purification of the product from the Mitsunobo reagents and byproducts is often challenging as well.

The present invention relates to 6C-purin-nucleoside derivatives. So far, the synthesis of 6C-purin-nucleoside derivatives with cross coupling has been limited because here, the substrate scope is limited to simple substituted aromatics. The advantage of the disclosed method is a wide variety of available possible modification groups which can be used for modification of the nucleoside.

Two general approaches have been identified to synthesize the claimed guanine nucleoside analogous.

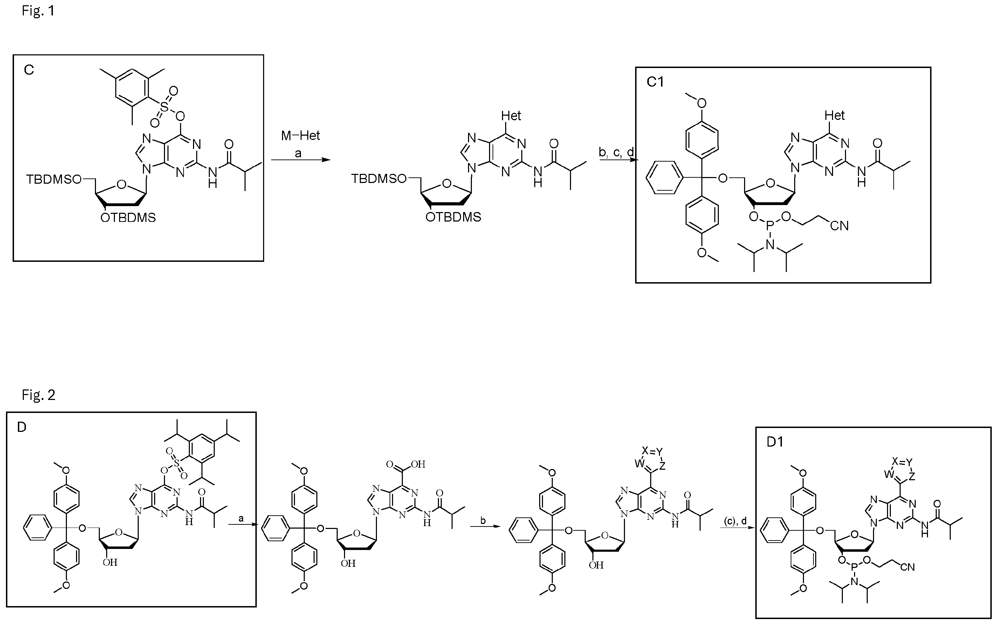

The first approach includes a key cross-coupling type reaction to directly achieve coupling of the purine nucleoside to the heterocycle (Fig. 1).

The second approach features an on-nucleoside formation of the desired heterocycle, all stemming from the common precursor, the carboxylic acid 6Ca, which is derived from a guanine-sulfonate via carbonylative cross coupling (Fig. 2). Furthermore, this approach grants access to a range of substituted amide compounds, which can be utilized for on-nucleic acid labeling.

### SUMMARY OF THE INVENTION

The invention relates to a method of synthesizing of 6C modified guanosine analogues, wherein
a) the guanosine analogue comprises 2'deoxy-ribose or ribose,
b) the guanosine analogue is protected at the 5' position by DMT group (dimethoxytrityl),
c) the guanosine analogue is phosphorylated at 3' position with (2-cyanoethoxy)(diisopropylamino)phosphaneyl-group,
d) the guanosine analogue is substituted at the amino group of 2C position with 2-methyl-1-oxopropyl group, and
e) the 6C modification of the guanosine analogue is selected from the group of
   a. a heterocycle (C1);
   b. a 5-membered heterocycle comprising W, X, Y and Z, wherein W, X, Y and Z can be C, N, O and S (D1);
   c. an oxadiazole (D2);
   d. a BOC-imidazole (B1) and
   e. BOC-pyrazole (B2);
wherein the method comprises the following steps:
i) providing a guanosine precursor molecule, wherein the guanosine precursor is protected by a first and a second protection group at the 3' and 5' positions, wherein the first and the second protection group are independently selected from:
   a. no protection group (A),
   b. a TBDMSO group ((1,1-dimethylethyl)dimethylsilyl) (C) and
   c. a DMT group (dimethoxytrityl) (D);
   and wherein the guanosine precursor molecule is protected by a third protection group at the 6C position, wherein the third protection group is selected from:
   a. no protection group (A),
   b. 2,4,6-trismethylbenzenesulfonate group (C)
   c. and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
   a. if the modification group is a heterocycle (C1) or BOC-pyrazole (B2) transfer is performed under conditions: presence of Pd(dba)₂, JohnPhos, K₃PO₄, 1,4-dioxane under reflux, or
   b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and BOC protection to form BOC-imidazole (B1),
iv) applying appropriate conditions for adding or removing protection groups to transform the guanosine precursor into 6C modified guanosine analogue, wherein step iv) may be performed at any time during synthesis of 6C modified guanosine analogue and may be performed more than once.

The invention relates to a 6C modified guanosine analogue, wherein
a) the guanosine analogue comprises 2'deoxy-ribose or ribose,
b) the guanosine analogue is protected at the 5' position by DMT group (dimethoxytrityl),
c) the guanosine analogue is phosphorylated at 3' position with (2-cyanoethoxy)(diisopropylamino)phosphaneyl-group,
d) the guanosine analogue is substituted at the amino group of 2C position with 2-methyl-1-oxopropyl group, and
e) the 6C modification of the guanosine analogue is selected from the group of a heterocycle (C1); a 5-membered heterocycle comprising W, X, Y and Z, wherein W, X, Y and Z can be C, N, O and S (D1); oxadiazole (D2); BOC-imidazole (B1) and BOC-pyrazole (B2).

The invention relates to a 6C modified guanosine analogue produced by the following method, wherein the method comprises the following steps:
i) providing a guanosine precursor molecule, wherein the guanosine precursor is protected by a first and a second protection group at the 3' and 5' positions, wherein the first and the second protection group are selected from no protection group (A), TBDMSO group ((1,1-dimethylethyl)dimethylsilyl) (C) and DMT group (dimethoxytrityl) (D); and wherein the guanosine precursor molecule is protected by a third protection group at the 6C position, wherein the third protection group is selected from no protection group (A), 2,4,6-trismethylbenzenesulfonate group (C) and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
   a. if the modification group is a heterocycle (C1) or BOC-pyrazole (B2) transfer is performed under conditions: presence of Pd(dba)₂, JohnPhos, K₃PO₄, 1,4-dioxane under reflux, or
   b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and BOC protection to form BOC-imidazole (B1),
iv) applying appropriate conditions for adding or removing protection groups to transform the guanosine precursor into 6C modified guanosine analogue, wherein step iv) may be performed at any time during synthesis of 6C modified guanosine analogue and may be performed more than once.

In an alternative embodiment the invention further relates to a method of synthesizing of 6C modified purine nucleoside analogues, wherein
a) the purine nucleoside analogue comprises 2'deoxy-ribose or ribose,
b) the purine nucleoside analogue is protected at the 5' position by DMT group (dimethoxytrityl),
c) the purine nucleoside analogue is phosphorylated at 3' position with (2-cyanoethoxy)(diisopropylamino)phosphaneyl-group,
d) the 6C modification of the purine nucleoside analogue is selected from the group of
   a. a heterocycle (C1);
   b. a 5-membered heterocycle comprising W, X, Y and Z, wherein W, X, Y and Z can be C, N, O and S (D1);
   c. an oxadiazole (D2);
   d. a BOC-imidazole (B1) and
   e. BOC-pyrazole (B2);
wherein the method comprises the following steps:
i) providing a purine nucleoside precursor molecule, wherein the purine nucleoside precursor is protected by a first and a second protection group at the 3' and 5' positions, wherein the first and the second protection group are independently selected from:
   a. no protection group (A),
   b. a TBDMSO group ((1,1-dimethylethyl)dimethylsilyl) (C) and
   c. a DMT group (dimethoxytrityl) (D);
   and wherein the purine nucleoside precursor molecule is protected by a third protection group at the 6C position, wherein the third protection group is selected from:
   a. no protection group (A),
   b. 2,4,6-trismethylbenzenesulfonate group (C)
   c. and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
   a. if the modification group is a heterocycle (C1) or BOC-pyrazole (B2) transfer is performed under conditions: presence of Pd(dba)₂, JohnPhos, K₃PO₄, 1,4-dioxane under reflux, or
   b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and BOC protection to form BOC-imidazole (B1),
iv) applying appropriate conditions for adding or removing protection groups to transform the purine nucleoside precursor into 6C modified purine nucleoside analogue, wherein step iv) may be performed at any time during synthesis of 6C modified purine nucleoside analogue and may be performed more than once.

In another embodiment the invention also relates to a 6C modified purine nucleoside analogue, wherein
a) the purine nucleoside analogue comprises 2'deoxy-ribose or ribose,
b) the purine nucleoside analogue is protected at the 5' position by DMT group (dimethoxytrityl),
c) the purine nucleoside analogue is phosphorylated at 3' position with (2-cyanoethoxy)(diisopropylamino)phosphaneyl-group,
d) the 6C modification of the purine nucleoside analogue is selected from the group of
   a. a heterocycle (C1);
   b. a 5-membered heterocycle comprising W, X, Y and Z, wherein W, X, Y and Z can be C, N, O and S (D1);
   c. an oxadiazole (D2);
   d. a BOC-imidazole (B1);
   e. BOC-pyrazole (B2); and
   f. an amide substituted with H, Me, Et, EtNH₂, propargyl or other side chains, especially containing amines or alkynes (E1)..

### BRIEF DESCRIPTION OF FIGURES

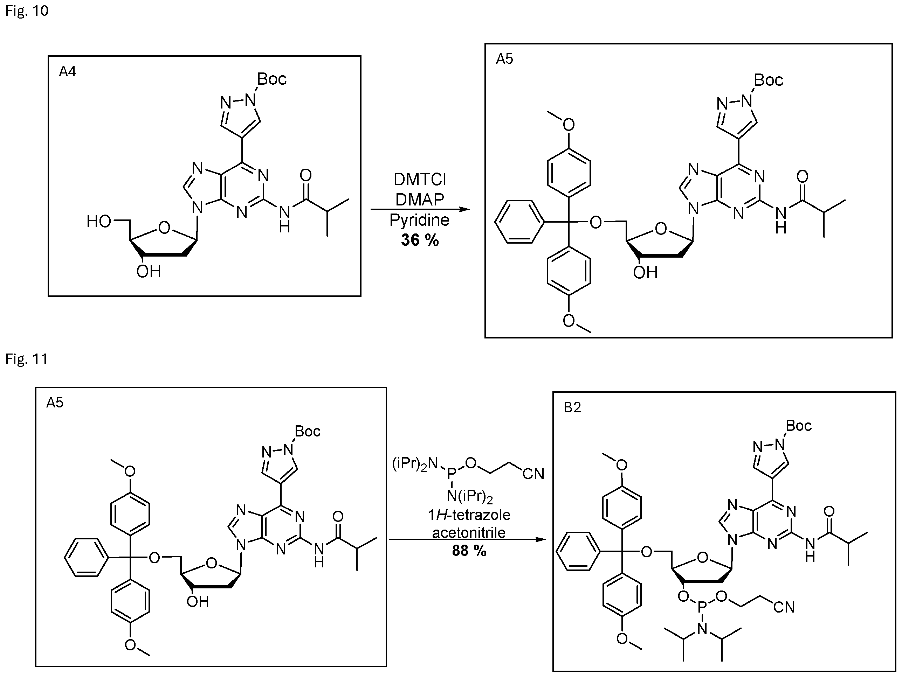

**Fig. 1**
   Cross-coupling type reaction to couple directly the purine nucleoside to the heterocycle: steps of synthesis are performed under the following conditions: step a): presence of Pd(dba)₂ ((Bis(dibenzylidenaceton)palladium)), JohnPhos (2-Biphenylyl)-di-*tert*.-butylphosphin), K₃PO₄, 1,4-dioxane under reflux, synthesis yield: 54%; step b): presence of TBAF (tetrabutylammonium fluoride), THF (tetrahydrofuran); step c): presence of DMTCI (4,4'dimethoxytrityl chloride), DMAP (4-dimethylaminopyridine), NEt₃ (triethylamine), pyridine; step d): 3-((bis(diisopropylamino)phosphaneyl)oxy)propanenitrile, 1H-tetrazole, acetonitrile. With Het being a heterocyclic structure and M being an organometallic compound, especially organoboronates such as B(OH)₂ or Bpin (bis(pinacolato)diboron).
**Fig. 2**
   Synthesis by carbonylative cross coupling includes the following steps a): presence of CO(g) or Co₂(CO)₈, KOAc, Pd(dppf)Cl₂ ((1,1'-Bis(diphenylphosphino)ferrocene]palladium(II) dichloride), EtOH; step b): HATU (1-[Bis(dimethylamino)methylene]-1H-1,2,3-triazolo[4,5-b]pyridinium 3-oxide hexafluorophosphate), DIPEA (N,N-diisopropylethylamine), DMF (dimethylformamide); step d): 3-((bis(diisopropylamino)phosphaneyl)oxy)propanenitrile, 1H-tetrazole, acetonitrile. Where W, X, Y, Z can be C, N, O, S, depending on the nucleophile and additive reagents the carboxylic acid obtained in step a) is condensed with in step b). Depending on the resulting 5-membered heterocycle, an additional protection step may be necessary to protect free NH-groups. The BOC-protection group is generally preferred for DNA-synthesis.
**Fig. 3**
   Synthesis by cross-coupling starts with the sulfonate. Overview of the synthetic approach towards PYR from previously published sulfonate precursor 9-((2R,4S,5R)-4-((tert-butyldimethylsilyl)oxy)-5-(((tert-butyldimethylsilyl)oxy)methyl)tetrahydrofuran-2-yl)-2-isobutyramido-9H-purin-6-yl 2,4,6-trimethylbenzenesulfonate: step a): presence of tert-butyl 4-(4,4,5,5-tetramethyl-1,3,2-dioxaborolan-2-yl)-1H-pyrazole-1-carboxylate 2.0 eq., Pd(dba)₂ 0.1 eq. ((Bis(dibenzylidenaceton)palladium)), JohnPhos (2-Biphenylyl)-di-*tert*.-butylphosphin), K₃PO₄, 1,4-dioxane under reflux, for 10 h, synthesis yield: 54%; b) presence of TBAF (tetrabutylammonium fluoride), THF (tetrahydrofuran), 0°C, 10 min. 77%; step c): presence of DMTCI (4,4'dimethoxytrityl chloride) 1.2 eq., DMAP (4-dimethylaminopyridine) 0.3 eq., NEt₃ (triethylamine) 2.0 eq., pyridine, at room temperature for 18 h, synthesis yield: 36%; step d): presence of 3-((bis(diisopropylamino)phosphaneyl)oxy)propanenitrile 1.05 eq., 1H-tetrazole 1.0 eq., acetonitrile, room temperature, 3 h, synthesis yield: 88%.
**Fig. 4**
   Overview of the synthetic approach towards IMI (imidazole - B1 compound) from N-iBu-deoxyguanosine: step a): presence of CO(g) or Co₂(CO)₈ 0.5 eq., KOAc 3.0 eq., Pd(dppf)Cl₂ 0.1 eq. ((1,1'-Bis(diphenylphosphino)ferrocene]palladium(II) dichloride), EtOH, at room temperature, 14 h; step b): presence of HATU 3.0 eq. (1-[Bis(dimethylamino)methylene]-1H-1,2,3-triazolo[4,5-b]pyridinium 3-oxide hexafluorophosphate), DIPEA 3.0 eq. (N,N-diisopropylethylamine), ethylenediamine 2.0 eq., DMF (dimethylformamide), at 60°C, for 14 h (10 % conversion by LC-MS data).
**Fig. 5**
   Overview of the synthetic approach towards 3DO (D2) from previously shown intermediate 9-((2R,4S,5R)-5-((bis(4-methoxyphenyl)(phenyl)methoxy)methyl)-4-hydroxytetrahydrofuran-2-yl)-2-isobutyramido-9H-purin-6-yl 2,4,6-triisopropylbenzenesulfonate (D): step a): presence of DMTCI 1.2 eq. (4,4'dimethoxytrityl chloride), DMAP 0.3 eq. (4-dimethylaminopyridine), NEt₃ 2.0 eq. (triethylamine), pyridine; step b): presence of TIPSCI 2.0 eq. (triispropylsilyl chloride), DMAP 0.35 eq., NEt₃ 2.0 eq., DCM (dichloromethane); step c): presence of CO(g) or Co₂(CO)₈ 0.5 eq., KOAc 3.0 eq., Pd(dppf)Cl₂ 0.1 eq. ((1,1'-Bis(diphenylphosphino)ferrocene]palladium(II) dichloride), EtOH; step d): presence of HATU 3.0 eq. (1-[Bis(dimethylamino)methylene]-1H-1,2,3-triazolo[4,5-b]pyridinium 3-oxide hexafluorophosphate), DIPEA 3.0 eq. (N,N-diisopropylethylamine), DMF (dimethylformamide), at 60°C, for 14 h (10 % conversion by LC-MS data).
**Fig. 5a**
   Overview of the synthetic approach towards 3DS (D3) from previously shown intermediate 9-((2R,4S,5R)-5-((bis(4-methoxyphenyl)(phenyl)methoxy)methyl)-4-hydroxytetrahydrofuran-2-yl)-2-isobutyramido-9H-purin-6-yl 2,4,6-triisopropylbenzenesulfonate (D): step a) presence of DMTCI 1.2 eq. (4,4'dimethoxytrityl chloride), DMAP 0.3 eq. (4-dimethylaminopyridine), NEt₃ 2.0 eq. (triethylamine), pyridine; step b): presence of TIPSCI 2.0 eq. (triispropylsilyl chloride), DMAP 0.35 eq., NEt₃ 2.0 eq., DCM (dichloromethane); step c): presence of CO(g) or Co₂(CO)₈ 0.5 eq., KOAc 3.0 eq., Pd(dppf)Cl₂ 0.1 eq. ((1,1'-Bis(diphenylphosphino)ferrocene]palladium(II) dichloride), EtOH; step d): presence of HATU 3.0 eq. (1-[Bis(dimethylamino)methylene]-1H-1,2,3-triazolo[4,5-b]pyridinium 3-oxide hexafluorophosphate), DIPEA 3.0 eq. (N,N-diisopropylethylamine), DMF (dimethylformamide), at 60°C, for 14 h (10 % conversion by LC-MS data); Lawessons reagent (2,4-Bis(4-methoxyphenyl)-1,3,2,4-dithiadiphosphetane-2,4-dithione), THF (tetrahydrofuran), under reflux.
**Fig. 6**
   Synthesis of 2'-deoxy-3',5'-bis-O-[(1,1-dimethylethyl)dimethylsilyl]-N- (2-methyl-1oxopropyl)guanosine (A1)
**Fig. 7**
   Synthesis of 9-((2R,4S,5R)-4-((tert-butyldimethylsilyl)oxy)-5-(((tert-butyldimethylsilyl)oxy)methyl)tetrahydrofuran-2-yl)-2-isobutyramido-9H-purin-6-yl 2,4,6-trimethylbenzenesulfonate (A2)
**Fig. 8**
   Synthesis of tert-butyl 4-(9-((2R,4S,5R)-4-((tert-butyldimethylsilyl)oxy)-5-(((tert-butyldimethylsilyl)oxy)methyl)tetrahydrofuran-2-yl)-2-isobutyramido-9H-purin-6-yl)-1H-pyrazole-1-carboxylate (A3)
**Fig. 9**
   Synthesis of tert-butyl 4-(9-((2R,4S,5R)-4-hydroxy-5-(hydroxymethyl)tetrahydrofuran-2-yl)-2-isobutyramido-9H-purin-6-yl)-1H-pyrazole-1-carboxylate (A4)
**Fig. 10**
   Synthesis of 2'-deoxy-5'-dimethoxytrityl-N-(2-methyl-1-oxopropyl)-, 6- [N-Boc-pyrazol]-guanosine (A5) (Fig. 10)
**Fig. 11**
   Synthesis of tert-butyl 4-(9-((2R,4S,5R)-5-((bis(4-methoxyphenyl)(phenyl)methoxy)methyl)-4-(((2-cyanoethoxy)(diisopropylamino)phosphaneyl)oxy)tetrahydrofuran-2-yl)-2-isobutyramido-9H-purin-6-yl)-1H-pyrazole-1-carboxylate (B2)
**Fig. 12**
   Overview of the synthetic approach towards substituted amide compounds from either key intermediates 6Ca or 9-((2R,4S,5R)-5-((bis(4-methoxyphenyl)(phenyl)methoxy)methyl)-4-hydroxytetrahydrofuran-2-yl)-2-isobutyramido-9H-purin-6-yl 2,4,6-triisopropylbenzenesulfonate.

R₁ can be R₂ can be H, Me, Et, EtNH₂, propargyl or other side chains, especially containing amines or alkynes for on-DNA labeling techniques. R₃ and R₄ can be tert-butyldimethylsilyl or R₃ can be dimethoxytrityl while R₄ is H

Step a): presence of CO(g) or Co₂(CO)₈ 0.5 eq., KOAc 3.0 eq., Pd(dppf)Cl₂ 0.1 eq ((1,1'-Bis(diphenylphosphino)ferrocene]palladium(II) dichloride), THF (tetrahydrofuran), NR₁R₂; step b): presence of NCR₁, KOAc, Pd(dppf)Cl₂, EtOH; step c): presence HATU (1-[Bis(dimethylamino)methylene]-1H-1,2,3-triazolo[4,5-b]pyridinium 3-oxide hexafluorophosphate), DIPEA (N,N-diisopropylethylamine), THF, NR₁R₂ (10 % conversion by LC-MS data)

### DETAILED DESCRIPTION OF THE INVENTION

The invention relates to a method of synthesizing of 6C modified guanosine analogues, wherein
a) the guanosine analogue comprises 2'deoxy-ribose or ribose,
b) the guanosine analogue is protected at the 5' position by DMT group (dimethoxytrityl),
c) the guanosine analogue is phosphorylated at 3' position with (2-cyanoethoxy)(diisopropylamino)phosphaneyl-group,
d) the guanosine analogue is substituted at the amino group of 2C position with 2-methyl-1-oxopropyl group, and
e) the 6C modification of the guanosine analogue is selected from the group of
   a. a heterocycle (C1);
   b. a 5-membered heterocycle comprising W, X, Y and Z, wherein W, X, Y and Z can be C, N, O and S (D1);
   c. an oxadiazole (D2);
   d. a BOC-imidazole (B1) and
   e. BOC-pyrazole (B2);
wherein the method comprises the following steps:
i) providing a guanosine precursor molecule, wherein the guanosine precursor is protected by a first and a second protection group at the 3' and 5' positions, wherein the first and the second protection group are independently selected from:
   a. no protection group (A),
   b. a TBDMSO group ((1,1-dimethylethyl)dimethylsilyl) (C) and
   c. a DMT group (dimethoxytrityl) (D);
   and wherein the guanosine precursor molecule is protected by a third protection group at the 6C position, wherein the third protection group is selected from:
   a. no protection group (A),
   b. 2,4,6-trismethylbenzenesulfonate group (C)
   c. and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
   a. if the modification group is a heterocycle (C1) or BOC-pyrazole (B2) transfer is performed under conditions: presence of Pd(dba)₂, JohnPhos, K₃PO₄, 1,4-dioxane under reflux, or
   b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and Boc protection to form BOC-imidazole (B1),
iv) applying appropriate conditions for adding or removing protection groups to transform the guanosine precursor into 6C modified guanosine analogue, wherein step iv) may be performed at any time during synthesis of 6C modified guanosine analogue and may be performed more than once.

The inventors surprisingly found that transfer of a wide variety of modification groups from the second precursor molecule to the guanosine precursor molecule can be achieved under optimized and specialized conditions. The conditions are described above in step iii) of the method of the invention. As shown in Fig. 1 step a, Fig. 3 step a and Fig. 8 transfer of the modification group may either be achieved in the presence of Pd(dba)₂ (Bis(dibenzylidenaceton)palladium), JohnPhos (2-Biphenylyl)-di-*tert*.-butylphosphin), K₃PO₄, 1,4-dioxane, which is performed under reflux. An experimental yield of 54% was achieved.

Transfer of the modification group is based on the experimental procedure of Lakshman et al. [1], 2'-deoxy-3',5'-bis-O-[(1,1-dimethylethyl)dimethylsilyl]-N-(2-methyl-1-oxopropyl)-, 6-[2,4,6-tris(1-methyl)benzenesulfonate]-guanosine 3 (A2) (450 mg, 0.60 mmol, 1.00 eq.), N-Boc-1H-pyrazole-4-boronic acid 19 (255.1 mg, 1.20 mmol, 2.00 eq.), potassium phosphate (255.4 mg, 1.20 mmol, 2.00 eq.), 2-(dicyclohexylphosphino)biphenyl (42.2 mg, 0.12 mmol, 0.20 eq.) and bis(dibenzylideneacetone)palladium(0) (17.3 mg, 0.03 mmol, 0.05 eq.) were dissolved in dry dioxane (9 ml). The reaction mixture was heated to 80°C (reflux) for 10-17 hours. Reaction time can be varied between 10-17 h, preferably reaction time is 11-17 h, more preferably reaction time is 12-17 h, even more preferably reaction time is 13-17 h, even more preferably reaction time is 14-17 h, even more preferably reaction time is 15-17 h, even more preferably reaction time is 16-17 h and most preferably reaction time is about 17 h. The reaction solution was then filtered over Celite and the solvent was removed from the filtrate under reduced pressure. The crude product was loaded on silica and purified by column chromatography (cyclohexane/ethyl acetate 2:1) to give 185 mg 2'-deoxy-3',5'-bis-O-[(1,1-dimethylethyl)dimethylsilyl]-N-(2-methyl-1-oxopropyl)-, 6-[N-Boc-pyrazole]-guanosine (A3) (185 mg, 0.26 mmol, 43%) was obtained as a colorless solid.

Throughout this disclosure amounts of educts for each of the reactions are provided in mass, molar amounts and relative equivalents (eq.) which indicate the relative amount of each of the compounds to each other.

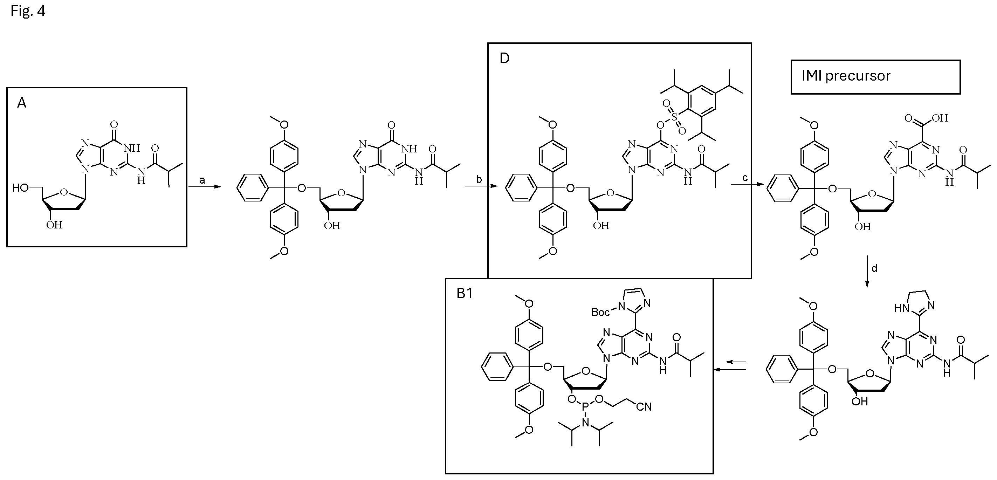

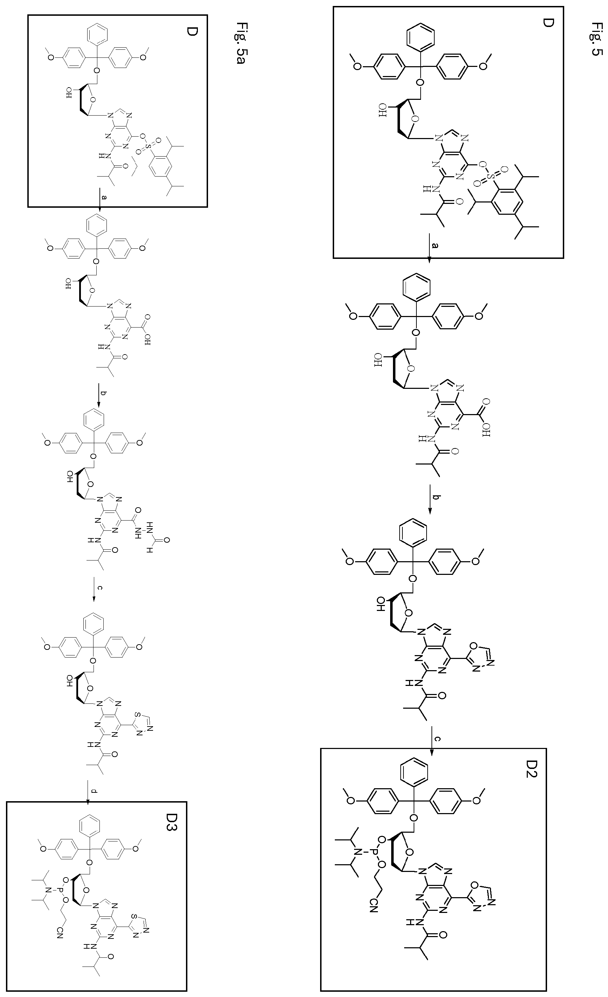

Alternatively, the transfer of the modification group may be achieved under conditions as shown in Fig. 2 step b, Fig. 4 step d, Fig. 5 step d and Fig. 5a step b: in the presence of HATU (1-[Bis(dimethylamino)methylene]-1H-1,2,3-triazolo[4,5-b]pyridinium 3-oxide hexafluorophosphate), DIPEA (N,N-diisopropylethylamine) and DMF (dimethylformamide).

Some steps of the method of the invention may be performed under an inert atmosphere. The gases of an inert atmosphere may be selected from the group consisting of argon and nitrogen, preferably argon is selected for inert atmosphere.

The transfer reaction or any other step is performed in a dry (water-free) organic solvent. Possible solvents for the method of this invention are selected from the group consisting of DCM (dichloromethane), THF (tetrahydrofuran), dioxane, DMF (dimethylformamide), MeOH (methanol) and EtOH (ethanol), MeCN (acetonitrile) preferably the solvent for transfer of the modification group is dioxane, more preferably the solvent is 1,4-dioxane.

The obtained modified guanosine nucleotide analogue may be subsequently incorporated into an oligonucleotide sequence. In incorporation may be achieved by solid-phase organic synthesis (SPOS).

The used precursors, the final product and intermediate products of the method may be protected by selected protection groups at least at the 3' and 5' positions or other reactive positions, in particular the 6C position suitable protection groups are selected from group consisting of a TBDMSO group ((1,1-dimethylethyl)dimethylsilyl), a DMT group (dimethoxytrityl), a 2,4,6-trismethylbenzenesulfonate group, a BOC group (tert-butyloxycarbonyl), a 2,4,6-tris(1-methylethyl)benzenesulfonate group, a PAC group (phenoxyacetyl) and an isobutyryl group.

Further, the invention relates to a 6C modified guanosine analogue, wherein
a) the guanosine analogue comprises 2'deoxy-ribose or ribose,
b) the guanosine analogue is protected at the 5' position by DMT group (dimethoxytrityl),
c) the guanosine analogue is phosphorylated at 3' position with (2-cyanoethoxy)(diisopropylamino)phosphaneyl-group,
d) the guanosine analogue is substituted at the amino group of 2C position with 2-methyl-1-oxopropyl group, and
e) the 6C modification of the guanosine analogue is selected from the group of a heterocycle (C1); a 5-membered heterocycle comprising W, X, Y and Z, wherein W, X, Y and Z can be C, N, O and S (D1); oxadiazole (D2); BOC-imidazole (B1) and BOC-pyrazole (B2).

The invention relates to a 6C modified guanosine analogue produced by the above described method under the described conditions, wherein the method comprises the following steps:
i) providing a guanosine precursor molecule, wherein the guanosine precursor is protected by a first and a second protection group at the 3' and 5' positions, wherein the first and the second protection group are independently selected from no protection group (A), TBDMSO group ((1,1-dimethylethyl)dimethylsilyl) (C) and DMT group (dimethoxytrityl) (D); and wherein the guanosine precursor molecule is protected by a third protection group at the 6C position, wherein the third protection group is selected from no protection group (A), 2,4,6-trismethylbenzenesulfonate group (C) and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
   a. if the modification group is a heterocycle (C1) or BOC-pyrazole (B) transfer is performed under conditions: presence of Pd(dba)₂, JohnPhos, K₃PO₄, 1,4-dioxane under reflux, or
   b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and BOC protection to form BOC-imidazole (B1),
iv) applying appropriate conditions for adding or removing protection groups to transform the guanosine precursor into 6C modified guanosine analogue, wherein step iv) may be performed at any time during synthesis of 6C modified guanosine analogue and may be performed more than once.

The method of the invention is not limited to guanosine analogues and can be used to obtain modified purine nucleoside analogues.

In an alternative embodiment the invention further relates to a method of synthesizing of 6C modified purine nucleoside analogues, wherein
a) the purine nucleoside analogue comprises 2'deoxy-ribose or ribose,
b) the purine nucleoside analogue is protected at the 5' position by DMT group (dimethoxytrityl),
c) the purine nucleoside analogue is phosphorylated at 3' position with (2-cyanoethoxy)(diisopropylamino)phosphaneyl-group,
d) the 6C modification of the purine nucleoside analogue is selected from the group of
   a. a heterocycle (C1);
   b. a 5-membered heterocycle comprising W, X, Y and Z, wherein W, X, Y and Z can be C, N, O and S (D1);
   c. an oxadiazole (D2);
   d. a BOC-imidazole (B1) and
   e. BOC-pyrazole (B2);
wherein the method comprises the following steps:
i) providing a purine nucleoside precursor molecule, wherein the purine nucleoside precursor is protected by a first and a second protection group at the 3' and 5' positions, wherein the first and the second protection group are independently selected from:
   a. no protection group (A),
   b. a TBDMSO group ((1,1-dimethylethyl)dimethylsilyl) (C) and
   c. a DMT group (dimethoxytrityl) (D);
   and wherein the purine nucleoside precursor molecule is protected by a third protection group at the 6C position, wherein the third protection group is selected from:
   d. no protection group (A),
   e. 2,4,6-trismethylbenzenesulfonate group (C)
   f. and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
   a. if the modification group is a heterocycle (C1) or BOC-pyrazole (B2) transfer is performed under conditions: presence of Pd(dba)₂, JohnPhos, K₃PO₄, 1,4-dioxane under reflux, or
   b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and BOC protection to form BOC-imidazole (B1),
iv) applying appropriate conditions for adding or removing protection groups to transform the purine nucleoside precursor into 6C modified purine nucleoside analogue, wherein step iv) may be performed at any time during synthesis of 6C modified purine nucleoside analogue and may be performed more than once.

Some steps of the method of the invention may be performed under an inert atmosphere. The gases of an inert atmosphere may be selected from the group consisting of Argon and nitrogen, preferably the argon is selected for inert atmosphere.

The transfer reaction or any other step is performed in a dry (water-free) organic solvent. Possible solvents for the method of this invention are selected from the group consisting of DCM (dichloromethane), THF (tetrahydrofuran), dioxane, DMF (dimethylformamide), MeOH (methanol) and EtOH (ethanol), acetonitrile (MeCN) preferably the solvent for transfer of the modification group is dioxane, more preferably the solvent is 1,4-dioxane.

The obtained modified guanosine nucleotide analogue may be subsequently incorporated into an oligonucleotide sequence. In incorporation may be achieved by solid-phase organic synthesis (SPOS).

In another embodiment the invention also relates to a 6C modified purine nucleoside analogue, wherein
a) the purine nucleoside analogue comprises 2'deoxy-ribose or ribose,
b) the purine nucleoside analogue is protected at the 5' position by DMT group (dimethoxytrityl),
c) the purine nucleoside analogue is phosphorylated at 3' position with (2-cyanoethoxy)(diisopropylamino)phosphaneyl-group,
d) the 6C modification of the purine nucleoside analogue is selected from the group of
   a. a heterocycle (C1);
   b. a 5-membered heterocycle comprising W, X, Y and Z, wherein W, X, Y and Z can be C, N, O and S (D1);
   c. an oxadiazole (D2);
   d. a BOC-imidazole (B1);
   e. BOC-pyrazole (B2); and
   f. an amide substituted with H, Me, Et, EtNH₂, propargyl or other side chains, especially containing amines or alkynes (E1).

The modified purine nucleoside analogue is obtained by above described method of synthesis.

The purine analogues may be based on a selection of purines. The purines may be selected from the group consisting of adenine, guanine, hypoxanthine, xanthine, theophylline, theobromine, caffeine, uric acid, isoguanine and inosine.

The present modified nucleotides may be incorporated into a nucleotide chain, i.e. a longer nucleic acid. Such nucleic acids may be between 200 and 5 nucleotides in length. They may be longer than 200 nucleotides, prepared by enzymatic ligation of nucleic acids between 200 and 5 nucleotides in length. The present nucleosides may be incorporated into nucleic acid probes of varying length, such as between 1000 nucleotides and 15 nucleotides, 100 nucleotides and 20 nucleotides and preferably between 50 nucleotides and 20 nucleotides and more preferably between 40 nucleotides and 15 nucleotides.

"Oligonucleotide or polynucleotide" generally refers to a polymer of single- or double-stranded nucleotides. As used herein, "oligonucleotide" and its grammatical equivalents will include the full range of nucleic acids. An oligonucleotide will typically refer to a nucleic acid molecule comprised of a linear strand of ribonucleotides. The exact size will depend on many factors, which in turn depends on the ultimate conditions of use, as is well known in the art.

In such a case where they are incorporated into a probe, or oligonucleotide, there may be one or more of such modified nucleosides present. That means there may only one modified nucleoside and the rest are standard nucleotides (A, C, G, T or U) or there may be multiple modified nucleosides present in the oligonucleotide (or probe).

In one embodiment there may different, i.e. a mixture of the claimed modified nucleosides present in the probe or oligonucleotide.

The present nucleosides may be used in catalytic DNA, such as but not limited to deoxyribozymes.

The present invention therefore relates to the use of the modified nucleosides according to the invention in a nucleic acid encoding a catalytic core region of a catalytic deoxyribozyme (DNAzyme), wherein said catalytic core region comprises the following sequence SEQ ID NO. 1, wherein the number in parenthesis designates the position number, 5'-G (1), G (2), C (C3), T (4), A (5), G (6), C (7), T (8), A (9), C (10), A (11), A (12), C (13), G (14), A (15)-3' (SEQ ID NO. 1), and wherein said core region nucleic acid sequence comprises one or more of the following modified nucleosides according to the invention,
i. a nucleoside that increases activity,
ii. a nucleoside that reduces or abolishes activity,
iii. a nucleoside that influences target association and/or dissociation resulting in a changed, reduced or improved catalytic turnover,
iv. a nucleoside that increases target RNA selectivity,
v. a nucleoside that reduces target RNA selectivity,
vi. a nucleoside that reduces the antisense effect via reduced RNase recruitment to increase the DNAzyme precision in (cellular) applications,
vii. a nucleoside that promotes the antisense effect via enhanced RNase recruitment to increase the overall effect of DNAzyme treatment in (cellular) applications
viii. a nucleoside that increases affinity to, or reduces the need for, metal ions, including but not limited to Mg²⁺,
ix. a nucleoside that increases cellular life-time,
x. a nucleoside that decreases innate immune response,
xi. a nucleoside that increases innate immune response.

In a preferred embodiment of the present invention, the nucleosides according to the invention are used at position 5, 6, and/or 14 of the catalytic core region.

The invention relates to the use of the claimed nucleosides in a nucleic acid encoding a catalytic deoxyribozyme (DNAzyme) comprising,
a. a catalytic core region,
b. and, at least one flanking variable target recognition sequence, wherein the target recognition sequence is between 5 and 30 nucleotides in length, and
c. wherein said at least one flanking target recognition sequence can be either 5' or 3' of said catalytic core region.

Herein, the nucleosides according to the invention may be in the catalytic core region. Or maybe in the flanking variable target recognition sequence. In one embodiment, the nucleosides according to the invention are both in the catalytic core region as well as the flanking variable target recognition sequence.

In a further embodiment, the nucleosides of the invention may be used in the synthesis of a nucleic acid by means of a polymerase.

In a further embodiment than, nucleosides of the present invention may be used in nucleic acid sequencing.

### EXAMPLES

### Synthesis of modified nucleoside phosphoramidites

### Examples for the synthesis of nucleosides by a cross-coupling approach

### Synthesis of PYR

After synthesis of common precursor sulfonated guanosine derivative (C), synthesis of the PYR nucleoside was achieved by Suzuki-coupling with the respective pinacolboronate tert-butyl 4-(4,4,5,5-tetramethyl-1,3,2-dioxaborolan-2-yl)-1H-pyrazole-1-carboxylate a. After fluoride mediated silyl deprotection (step b), the free PYR nucleoside was DMT protected (step c) and phosphorylated under standard conditions (step d).

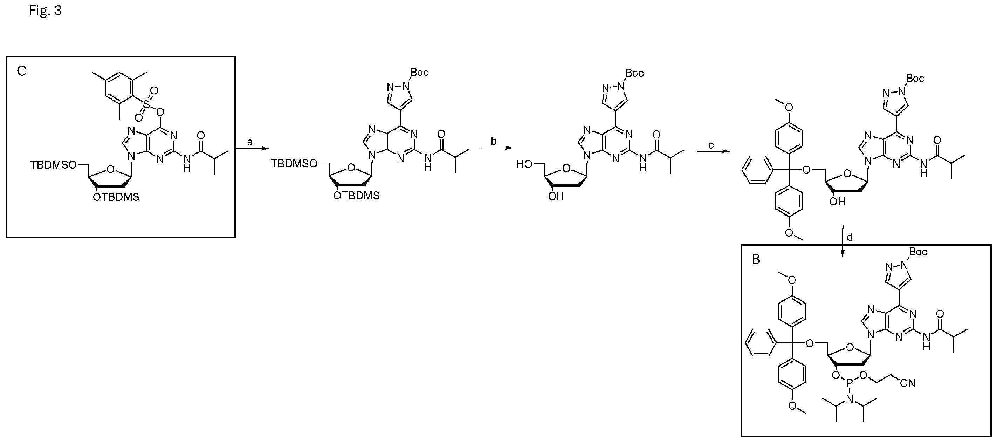

Synthesis of the respective phosphoramidite is accessible by standard methods as previously mentioned. Incorporation into an oligonucleotide by SPOS (solid-phase organic synthesis) is possible under standard conditions (see Fig. 3).

### Examples for the synthesis of Nucleosides via the carboxylic acid intermediate 6Ca

### Synthesis of IMI

After synthesis of common precursor 6Ca derivative (Fig. 4 and 5 D), synthesis of the IMI nucleoside precursor (as shown in Fig. 4) is achieved by condensation with ethylendiamine (as confirmed by LC-MS data).

For the synthesis of the respective phosphoramidite several steps are still needed, including oxidation of the imidazoline, BOC protection and 3'-phosphorylation. Incorporation into an oligonucleotide by SPOS is possible under standard conditions (see Fig. 4).

### Synthesis of 3DO

After synthesis of the key precursor 6Ca derivative (D), synthesis of the 3DO DMT (dimethoxytrityl)-protected nucleoside (D2) is achieved by condensation with formylhydrazine (step a).

Synthesis of the respective phosphoramidite is accessible by standard methods as previously mentioned. Incorporation into an oligonucleotide by SPOS is possible under standard conditions (see Fig. 5).

### Synthesis of 3DS

After synthesis of the key precursor 6Ca derivative (D), synthesis of the 3DS DMT-protected nucleoside (D3) could be achieved in analogy to 3DO by a 2 step condensation with formylhydrazine and ring-closing in presence of Lawesson's Reagent.

Synthesis of the respective phosphoramidite is accessible by standard methods as previously mentioned. Incorporation into an oligonucleotide by SPOS is possible under standard conditions (see Fig 5a).

### Chemical synthesis

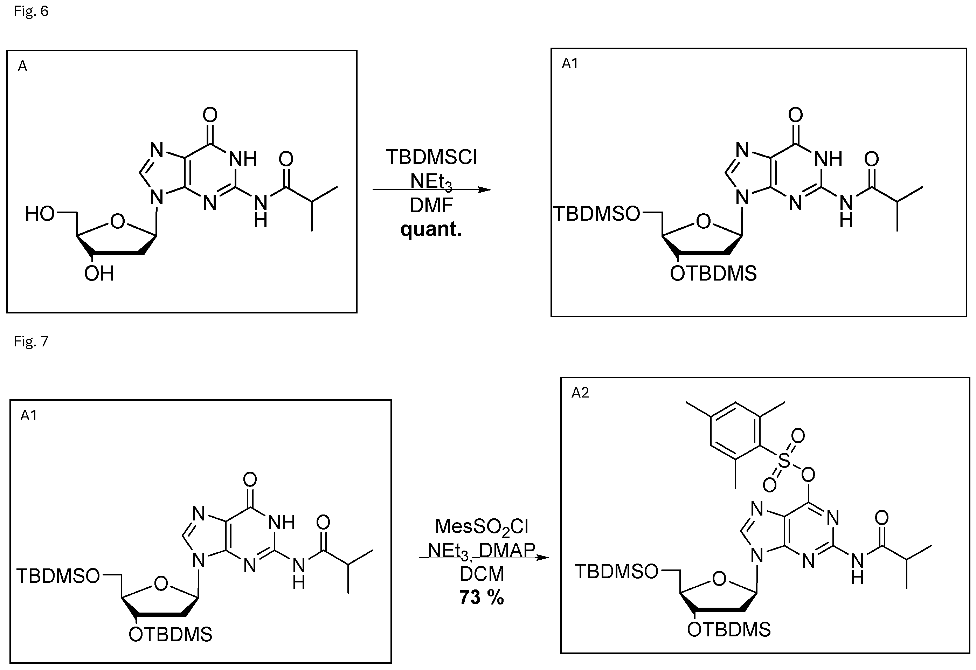

### Synthesis of 2'-deoxy-3',5'-bis-O-[(1,1-dimethylethyl)dimethylsilyl]-N- (2-methyl-1oxopropyl)guanosine (A1) (Fig. 6)

Based on Westmore and coworkers [2], 2'-deoxy-N-(2-methyl-1-oxopropyl)guanosine 1 A (7.00 g, 20.75 mmol, 1.0 eq.) was dissolved in DMF (dimethylformamide) (70 ml) under argon atmosphere. The reaction mixture was then cooled to 0°C and triethylamine (13.00 ml, 93.38 mmol, 4.5 eq.) and TBDMSCI (tert-butyldimethyl silyl chloride) (12.51 g, 83.00 mmol, 4.0 eq.) were added. The reaction solution was stirred for 16 hours and slowly warmed to room temperature. After complete conversion, the solution was diluted with ethyl acetate (3x40 ml) and washed with water (2x20 ml) and saturated sodium chloride solution (40 ml). The combined organic phases were then dried over sodium sulfate. The solvent was removed under reduced pressure. The crude product was mounted on silica and purified by column chromatography on silica gel (DCM:MeOH (dichloromethane:methanol) = 20:1 → DCM/MeOH 10:1). It yielded 11.95 g of 2'-deoxy-3',5'-bis-O-[(1,1-dimethylethyl)dimethylsilyl]- N-(2-methyl-1-oxopropyl)guanosine 2 (A1). The yield of the reaction was 11.95 g, 21.12 mmol, 100% as a yellowish solid (M (C₂₆H₄₇N₅O₅Si₂): 565.31 g/mol).

Characterization of the product provided the following measurements:
Yield: 11.95 g, 21.12 mmol, 100%.

Rf-value: 0.23 (dichloromethane/methanol 10:1) as determined by thin layer chromatography (TLC).

1H-NMR: (500 MHz, CDCl3) δ [ppm] = 11.96 (s, 1H, H-8), 8.37 (s, J = 3.4 Hz, 1H, H-18), 7.95 (s, 1H, H-1), 6.22 (t, J = 6.5 Hz, 1H, H-10), 4.57 (dt, J = 5.7, 3.5 Hz, 1H, H-12), 3.97 (q, J = 3.4 Hz, 1H, H-13), 3.75 (dd, J = 3.5,1.5 Hz, 2H, H-15, H-15'), 2.62 (p, J = 6.9 Hz, 1H, H-20), 2.50 - 2.41(m, 1H, H-14), 2.35 (ddd, J = 13.1, 6.1, 3.7 Hz, 1H, H-14'), 1.27 (dd,J = 6.9, 5.0 Hz, 6H, H-21, H-22), 0.90 (d, J = 4.0 Hz, 18H, H-28, H-29,H-30, H-35, H-36, H-37), 0.10 (s, 6H, H-32, H-33), 0.07 (d, J = 3.4 Hz, 6H, H-25, H-26).

13C-NMR: (126 MHz, CDCl3) δ [ppm] = 178.10 (C19), 162.60 (DMF), 155.49 (C9),147.79 (C4), 147.33 (C3), 136.72 (C1), 121.55 (C7), 87.99 (C13),83.52 (C10), 71.89 (C12), 62.80 (C15), 41.36 (C14), 36.63 (C20),36.52 (DMF), 31.47 (DMF), 25.97 (C28, C29, C30), 25.75 (C35, C36,C37), 19.01 und 18.98 (C21, C22), 18.44 (C27), 18.01 (C34), -4.67, -4.76 (C32, C33), -5.37, -5.50 (C24, C25).

HRMS (high resolution mass spectrometry) (ESI - electrospray ionization): was used to determine m/z = 566.31899 [M+H]⁺ and m/z = 588.30075 [M+Na]⁺.

FT-IR-ATR (Fourier transform infrared attenuated total reflectance spectroscopy): *ṽ*[cm-1] = 2955 (w), 2928 (w), 2857 (w), 1721 (m), 1680 (w), 1638 (m),1607 (m), 1553 (w), 1539 (w), 1387 (w), 1256 (m), 1138 (w), 1094 (m),1074 (m), 1061 (m), 1024 (m), 1007 (w), 935 (w), 885 (w), 835 (s),771 (s), 710 (w), 660 (w).

### Synthesis of 9-((2R,4S,5R)-4-((tert-butyldimethylsilyl)oxy)-5-(((tert-butyldimethylsilyl)oxy)methyl)tetrahydrofuran-2-yl)-2-isobutyramido-9H-purin-6-yl 2,4,6-trimethylbenzenesulfonate (A2) (Fig. 7)

2'-deoxy-3',5'-bis-O-[(1,1-dimethylethyl)dimethylsilyl]-N-(2-methyl-1-oxopropyl)guanosine 2 (A1) (1.00 g, 1.77 mmol, 1.00 eq.) was dissolved in DCM (51 ml) under argon atmosphere. Under cooling to 0°C, triethylamine (0.49 ml, 3.53 mmol, 2.00 eq.), 4-(dimethylamino)pyridine (0.08 g, 0.62 mmol, 0.35 eq.) and mesitylene-2-sulfonyl chloride (0.77 g, 3.53 mmol, 2.00 eq.) were added and stirred for 17 h. After complete conversion, the reaction solution was taken up in DCM and washed with saturated NaHCO₃ solution (10 ml) and saturated NaCl solution (15 ml). The combined organic phases were dried over magnesium sulphate. The solvent was removed under reduced pressure and the crude product obtained was loaded on silica and purified by column chromatography on silica gel (cHex/EtOAc 4:1). This yielded 955 mg of 9-((2R,4S,5R)-4-((tert-butyldimethylsilyl)oxy)-5-(((tert-butyldimethylsilyl)oxy)methyl)tetrahydrofuran-2-yl)-2-isobutyramido-9H-purin-6-yl 2,4,6-trimethylbenzenesulfonate (A2) (M (C₃₅H₅₇N₅O₇SSi₂): 747.35 g/mol).

The yield of the reaction was 955 mg,1.28 mmol, 72%, which was obtained as a colorless solid.

Characterization of the product provided the following measurements.

Yield: 955 mg, 1.28 mmol, 72%.

Rf-value: 0.3 (cyclohexane/ ethyl acetate 4:1), determined by TLC 1H-NMR: (300 MHz, CDCl3) δ [ppm] = 8.23 (s, 1H, H-1), 7.71 (s, 1H, H-18), 7.00(s, 2H, H-44, H-46), 6.40 (t, J = 6.4 Hz, 1H, H-10), 4.61 (dt, J = 6.1,3.4 Hz, 1H, H-12), 4.00 (q, J = 3.4 Hz, 1H, H-13), 3.86 (dd, J = 11.2,4.0 Hz, 1H, H-15), 3.76 (dd, J = 11.2, 3.3 Hz, 1H, H-15'), 3.10 (s, 1H,H-20), 2.74 (s, 6H, H-48, H-49), 2.61 (dt, J = 12.8, 6.3 Hz, 1H, H-14),2.41 (ddd, J = 13.1, 6.2, 3.7 Hz, 1H, H-14'), 2.33 (s, 3H, H-50), 1.22 (d,J = 6.9 Hz, 6H, H-21, H-22), 0.90 (d, J = 5.5 Hz, 18H, H-28, H-29, H-30,H-35, H-36, H-37), 0.10 (d, J = 2.4 Hz, 6H, H-32, H-33), 0.08 (s, 6H, H-25, H-26).

13C-NMR: (126 MHz, CDCl3) δ [ppm] = 154.62 (C4), 154.48 (C3), 151.13 (C9),144.29 (C45), 142.68 (C1), 140.40 (C43), 132.13 (C42), 131.85 (C46),119.59 (C7), 88.20 (C13), 84.68 (C10), 71.95 (C12), 62.86 (C15), 41.37(C14), 25.98 (C28, C29, C30), 25.76 (C35, C36, C37), 22.80 (C48, C49),21.17 (C50), 19.13 (C21, C22), 18.44 (C27), 17.99 (C34), -4.67und -4.78 (C32, C33), -5.35 und -5,47 (C24, C25).

HRMS (ESI): m/z = 770.34130 [M+Na]⁺.

FT-IR (ATR): *ṽ*[cm-1] = 2953 (w), 2930 (w), 2857 (w), 1722 (w), 1680 (w), 1618 (w),1578 (w), 1560 (w), 1508 (w), 1443 (w), 1402 (w), 1373 (m), 1362 (m), 1327 (w), 1254 (m), 1215 (w), 1198 (w), 1175 (m), 1140 (w), 1098 (m), 1072 (m), 1024 (m), 1007 (w), 937 (w), 883 (w), 833 (s), 814 (w), 775 (s), 723 (w), 662 (s), 644 (m).

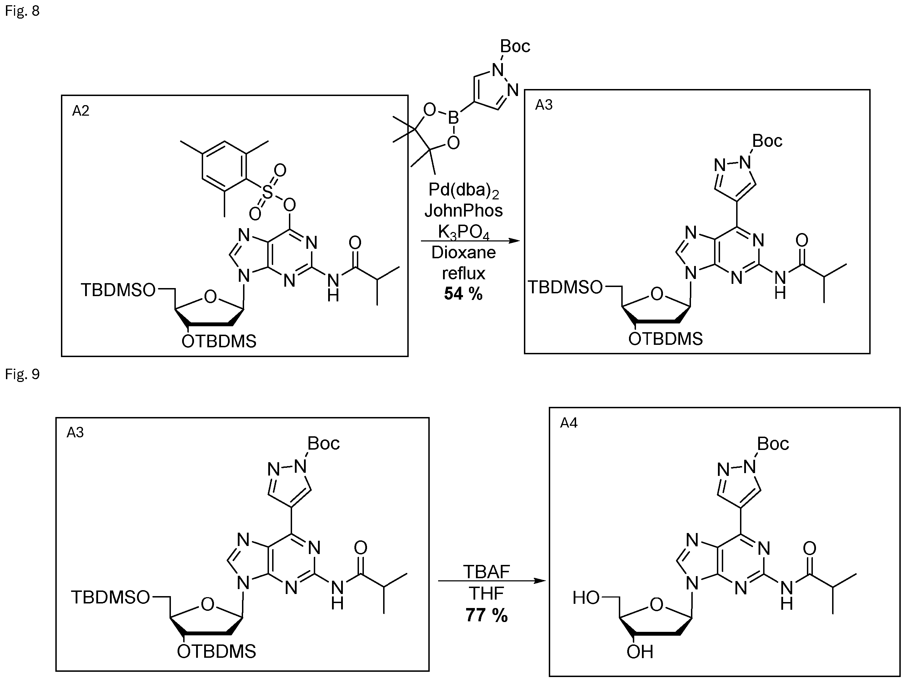

### Synthesis of tert-butyl 4-(9-((2R,4S,5R)-4-((tert-butyldimethylsilyl)oxy)-5-(((tert-butyldimethylsilyl)oxy)methyl)tetrahydrofuran-2-yl)-2-isobutyramido-9H-purin-6-yl)-1H-pyrazole-1-carboxylate (A3) (Fig. 8)

Based on the experimental procedure of Lakshman et al. [26], 2'-deoxy-3',5'-bis-O-[(1,1-dimethylethyl)dimethylsilyl]-N-(2-methyl-1-oxopropyl)-, 6-[2,4,6-tris(1-methyl)benzenesulfonate]-guanosine 3 (A2) (450 mg, 0.60 mmol, 1.00 eq.), N-Boc-1H-pyrazole-4-boronic acid 19 (255.1 mg, 1.20 mmol, 2.00 eq.), potassium phosphate (255.4 mg, 1.20 mmol, 2.00 eq.), 2-(dicyclohexylphosphino)biphenyl (42.2 mg, 0.12 mmol, 0.20 eq.) and bis(dibenzylideneacetone)palladium(0) (17.3 mg, 0.03 mmol, 0.05 eq.) were dissolved in dry dioxane (9 ml). The reaction mixture was heated to 80°C for 17 hours. The reaction solution was then filtered over Celite and the solvent was removed from the filtrate under reduced pressure. The crude product was loaded on silica and purified by column chromatography (cyclohexane/ethyl acetate 2:1) to give 185 mg 2'-deoxy-3',5'-bis-O-[(1,1-dimethylethyl)dimethylsilyl]-N-(2-methyl-1-oxopropyl)-, 6-[N-Boc-pyrazole]-guanosine (A3) (185 mg, 0.26 mmol, 43%) was obtained as a colorless solid (M (C₃₄H₅₇N₇O₆Si₂): 715.39 g/mol).

Characterization of the product provided the following measurements:
Yield: 185 mg, 0.26 mmol, 43%.

Rf-value: 0.49 (Cyclohexan/ Ethylacetat 2:1) determined by TLC.

1H-NMR: (500 MHz, CDCl3) δ [ppm] = 9.20 (s, 1H, H-40), 8.65 (s, 1H, H-38), 8.29(s, 1H, H-1), 8.02 (s, 1H, H-18), 6.45 (t, J = 6.5 Hz, 1H, H-10), 4.64 (dt,J = 6.0, 3.6 Hz, 1H, H-12), 4.02 (q, J = 3.5 Hz, 1H, H-13), 3.87 (dd,J = 11.2, 4.2 Hz, 1H, H-15), 3.79 (dd, J = 11.1, 3.4 Hz, 1H, H-15'), 3.24(s, 1H, H-20), 2.68 (dt, J = 12.8, 6.3 Hz, 1H, H-14), 2.45 (ddd, J = 13.1,6.1, 3.8 Hz, 1H, H-14'), 1.70 (s, 9H, H-47, H-48, H-49), 1.31 (d, J = 6.9Hz, 6H, H-21, H-22), 0.92 (d, J = 3.7 Hz, 18H, H-28, H-29, H-30, H-35,H-36, H-37), 0.12 (s, 6H, H-32, H-33), 0.09 (s, 6H, H-25, H-26).

13C-NMR: (126 MHz, CDCl3) δ [ppm] =152.54 (C4), 152.37 (C3), 143.73 (C38),132.94 (C40), 121.29 (C39), 88.04 (C13), 86.26 (C46), 84.27 (C10),71.92 (C12), 62.85 (C15), 41.17 (C14), 27.95 (C47, C48, C49), 25.99(C28, C29, C30), 25.78 (C35, C36, C37), 19.32 (C21, C22), 18.44 (C27),18.01 (C34), -4.65 und -4.76 (C32, C33), -5.34 und -5.45 (C24, C25).

HRMS (ESI): m/z = 716.39828 [M+H]⁺, m/z = 738.37996 [M+Na]⁺ as determined by mass spectroscopy.

FT-IR (ATR): *ṽ*[cm-1] = 2955 (w), 2928 (w), 2857 (w), 1753 (w), 1684 (w), 1603 (w),1537 (w), 1499 (w), 1462 (w), 1392 (m), 1360 (w), 1339 (w), 1287 (w),1252 (w), 1240 (m), 1213 (w), 1153 (w), 1128 (w), 1109 (w), 1094 (w),1072 (w), 1026 (w), 1007 (w), 949 (s), 887 (w), 833 (s), 812 (w), 800 (w),777 (s), 767 (m), 748 (w), 692 (w), 669 (w), 642 (w).

### Synthesis of tert-butyl 4-(9-((2R,4S,5R)-4-hydroxy-5-(hydroxymethyl)tetrahydrofuran-2-yl)-2-isobutyramido-9H-purin-6-yl)-1H-pyrazole-1-carboxylate (A4) (Fig. 9)

2'-deoxy-3',5'-bis-O-[(1,1-dimethylethyl)dimethylsilyl]-N-(2-methyl-1-oxopropyl)-, 6-[N- Boc-pyrazole]-guanosine (A3) (485 mg, 0.67 mmol, 1.0 eq.) was dissolved in dry THF (5.9 ml) under argon atmosphere. Under cooling to 0°C, TBAF (tetrabutylammonium fluoride) (1M in THF, 1.35 ml,1.35 mmol, 2.0 eq.) was added. The reaction mixture was stirred for 10 minutes. The solvent was then removed under reduced pressure. The crude product was purified by column chromatography on silica gel (DCM/MeOH 20:1) to afford 255 mg of 2'-deoxy-N-(2-methyl-1-oxopropyl)-, 6-[N-Boc-pyrazole]-guanosine 9 A4 (255 mg, 0.52 mmol, 77%) as a colorless solid (M (C₂₂H₂₉N₇O₆):487.22 g/mol).

Characterization of the product provided the following measurements:
Yield: 255 mg, 0.52 mmol, 77%.

Rf-value: 0.21 (dichloromethane/methanol 20:1) as determined by TLC.

1H-NMR: (500 MHz, CDCl3) δ [ppm] = 9.18 (s, 1H, H-26), 8.62 (s, 1H, H-24), 8.16(s, 1H, H-18), 8.10 (s, 1H, H-1), 6.41 (t, J = 6.8 Hz, 1H, H-10), 5.03 (s,1H, H-12), 4.21 - 4.15 (m, 1H, H-13), 3.97 (dd, J = 12.5, 2.6 Hz, 1H,H-15), 3.87 (dd, J = 12.4, 2.7 Hz, 1H, H-15'), 3.08 - 2.97 (m, 1H, H-20),2.91 (s, 1H, H-14), 2.46 (d, J = 13.6 Hz, 1H, H-14'), 1.70 (s, 9H, H-33,H-34, H-35), 1.32 (dd, J = 6.9, 2.0 Hz, 6H, H-21, H-22).

13C-NMR: (126 MHz, CDCl3) δ [ppm] = 152.03 (C4), 151.94 (C3), 147.17 (C29),143.65 (C24), 133.10 (C26), 121.01 (C25), 88.42 (C13), 86.44 (C32),86.13 (C10), 72.11 (C12), 62.74 (C15), 53.44 (DCM), 40.33 (C14), 27.94 (C33, C34, C35), 19.39 (C21, C22).

HRMS (ESI): m/z = 488.22449 [M+H]⁺, m/z = 510.20670 [M+Na]⁺ as determined by mass spectroscopy.

FT-IR (ATR): *ṽ*[cm-1] = 3347 (w), 2978 (w), 2926 (w), 2880 (w), 2602 (w), 2531 (w),2496 (w), 1597 (w), 1474 (m), 1443 (m), 1396 (m), 1366 (m), 1331 (w),1221 (w), 1184 (w), 1171 (w), 1159 (w), 1098 (w), 1072 (w), 1036 (s),1011 (w), 935 (w), 891 (w), 851 (w), 804 (s), 760 (w), 698 (w), 646 (w),638 (w).

### Synthesis of 2'-deoxy-5'-dimethoxytrityl-N-(2-methyl-1-oxopropyl)-, 6- [N-Boc-pyrazol]-guanosine (A5) (Fig. 10)

2'-deoxy-N-(2-methyl-1-oxopropyl)-, 6-[N-Boc-pyrazol]-guanosin 9 (A4) (10.3 mg, 0.021 mmol, 1.0 eq.), triethylamine (5.89 µl, 0.042 mmol, 2.0 eq.) and 2-(dimethylamino)pyridine (0.77 mg, 0.006 mmol, 0.3 eq.) were dissolved in dry pyridine (0.22 ml) under argon atmosphere. Dimethoxytrityl chloride (8.7 mg, 0.025 mmol, 1.22 eq.) was then added and the reaction solution was stirred for 18 hours at room temperature. The solvent was removed under reduced pressure. The crude product obtained was purified by column chromatography on silica gel (DCM/MeOH/TEA 20:1:0.2 - dichloromethane/methanol/triethylamine). It yielded 6 mg of 2'-deoxy-5'-dimethoxytrityl-N-(2-methyl-1-oxopropyl)-, 6- [N-Boc-pyrazole]-guanosine (A5) (6 mg, 0.007 mmol, 36%) as a yellowish solid (M (C₄₃H₄₇N₇O₈): 789,35 g/mol).

Characterization of the product provided the following measurements:
Yield: 6 mg, 0.007 mmol, 36%.

Rf-value: 0.4 (DCM:Methanol = 20:1 + 1% v/v NEt₃ (triethylamine), determined by TLC.

1H-NMR: (500 MHz, CDCl3) δ [ppm] = 9.19 (s, 1H, H-26), 8.64 (s, 1H, H-24), 8.12(s, 1H, H-1), 8.02 (s, 1H, H-18), 7.38 (d, J = 7.3 Hz, 2H) und 7.29 (s, 3H) und 7.22 (t, J = 7.4 Hz, 2H), 7.17 (dd, J = 7.9, 5.6 Hz, 2H) and 7.12 (d, J = 9.0 Hz, 1H) (H-38, H-39, H-41, H-42, H-50, H-51, H-53, H-54), 6.84 (dd, J = 11.5, 8.5 Hz, 2H) und 6.80 - 6.74 (m, 5H, H-45 - H-49), 6.52 (t, J = 6.4 Hz, 1H, H-10), 4.80 (s, 1H, H-12), 4.18 (d, J = 4.3 Hz, 1H, H-13), 3.81 (s, 1H, H-36), 3.75 (d, J = 1.4 Hz, 6H, H-56, H-58), 3.43 (dd,J = 10.2, 4.9 Hz, 1H, H-15), 3.37 (dd, J = 10.2, 4.1 Hz, 1H, H-15'), 2.84 (dt, J = 13.0, 6.3 Hz, 1H, H-14), 2.65 - 2.59 (m, 1H, H-14'), 1.70 (s, 9H, H-33, H-34, H-35), 1.27 (s, 6H, H-21, H-22).

13C-NMR: (126 MHz, CDCl3) δ [ppm] = 158.91 (C40, C52), 144.60 (C37, C43),143.55 (C24), 142.39 (C1), 132.81 (C26), 130.07 und 130.03 und 129.14 und 128.12 und 127.86 (C38, C39, C41, C42, C44, C50, C51, C53, C54), 121.16 (C25), 113.15 (C45-C49), 86.32 (C32), 86.25 (C13) 84.22 (C10),72.28 (C12), 63.87 (C15), 55.20 (C56, C58), 45.92 (Impurities at 3.14 ppm in 1H), 40.35 (C14), 27.95 (C33, C34, C35), 19.34 (C21, C22).

HRMS (ESI): m/z = 790.35717 [M+H]⁺, m/z = 812.33870 [M+Na]⁺ as determined by mass spectroscopy.

### Synthesis of tert-butyl 4-(9-((2R,4S,5R)-5-((bis(4-methoxyphenyl)(phenyl)methoxy)methyl)-4-(((2-cyanoethoxy)(diisopropylamino)phosphaneyl)oxy)tetrahydrofuran-2-yl)-2-isobutyramido-9H-purin-6-yl)-1H-pyrazole-1-carboxylate (B2) (Fig. 11)

Tert-butyl 4-(9-((2R,4S,5R)-5-((bis(4-methoxyphenyl)(phenyl)methoxy)methyl)-4-hydroxytetrahydrofuran-2-yl)-2-isobutyramido-9H-purin-6-yl)-1H-pyrazole-1-carboxylate (A5) (190 mg, 241 µmol, 1.00 eq.) was coevaporated two times with dry acetonitrile (5 mL). The resulting solid was redissolved in DCM (2.0 mL) under inert atmosphere and a freshly dried solution of 1H-tetrazole (16.9 mg, 535 µL, 241 µmol, 0.45M, 1.00 eq.) in acetonitrile was added.3-bis[di(propan-2-yl)amino]phosphanyloxypropanenitrile (76.1 mg, 80.2 µL, 253 µmol, 1.05 eq.) was added and the solution was stirred for 2 h at room temperature. The reaction mixture was diluted with DCM (10 mL), washed twice with saturated NaHCO₃ solution, dried over magnesium sulphate and the solvent was evaporated under reduced pressure at 30°C. The crude product was purified by column chromatography over silica (4:1 = cyclohexane:acetone + 1% v/v 7M NH3 in MeOH) and tert-butyl 4-(9-((2R,4S,5R)-5-((bis(4-methoxyphenyl)(phenyl)methoxy)methyl)-4-(((2-cyanoethoxy)(diisopropylamino)phosphaneyl)oxy)tetrahydrofuran-2-yl)-2-isobutyramido-9H-purin-6-yl)-1H-pyrazole-1-carboxylate (B2) (210 mg, 212 µmol, 88% yield) was obtained as an off-white foam.

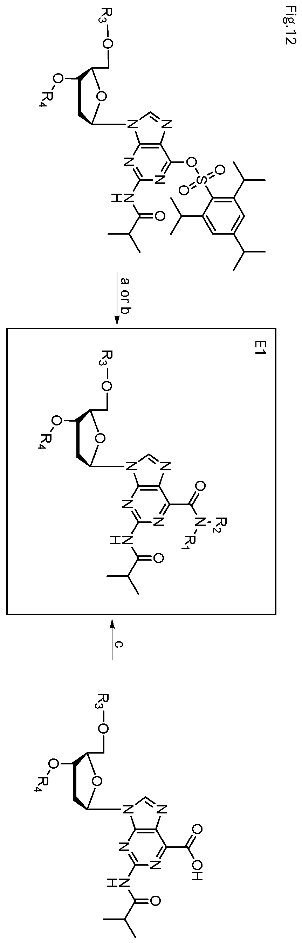

### Synthesis of 9-((2R,4S,5R)-4-((tert-butyldimethylsilyl)oxy)-5-(((tert-butyldimethylsilyl)oxy)methyl)tetrahydrofuran-2-yl)-N,N-diethyl-2-isobutyramido-9H-purine-6-carboxamide (Fig. 12)

Under Ar-atmosphere, 9-((2R,4S,5R)-4-((tert-butyldimethylsilyl)oxy)-5-(((tert-butyldimethylsilyl)oxy)methyl)tetrahydrofuran-2-yl)-2-isobutyramido-9H-purin-6-yl 2,4,6-trimethylbenzenesulfonate (10.0 mg, 13.4 µmol, 1.00 eq.), Co₂(CO)₈ (2.30 mg, 1.23 µL, 6.68 µmol, 0.500 eq.), Pd(dppf)Cl₂ (978 µg, 1.34 µmol, 0.100 eq.) and KOAc (3.94 mg, 40.1 µmol, 3.00 eq.) were dissolved in 0.4 mL THF. Ethylendiamine (1.47 mg, 2.07 µL, 20.1 µmol, 1.50 eq.) was added and the mixture was heated to 60 °C and stirred for 14 h. The reaction mixture was cooled, evaporated under reduced pressure and purified by flash-column chromatography with a linear gradient (0-5% MeOH in DCM).

### Reference list

[1] Lakshman, M. K., Gunda, P., & Pradhan, P. (2005). Mild and room temperature C-C bond forming reactions of nucleoside C-6 arylsulfonates. Journal of Organic Chemistry, 70(25), 10329-10335. https://doi.org/10.1021/jo0513764
[2.] Ogilvie, K. K., Sadana, K. L., Thompson, E. A., Quilliam, M. A., & Westmore, J. B. (1974). The use of silyl groups in protecting the hydroxyl functions of ribonucleosides. Tetrahedron Letters, 15(33), 2861-2863. https://doi.org/10.1016/S0040-4039(01)91763-0

### Abbreviation list

BOC: tert-butyloxycarbonyl
Bpin: bis(pinacolato)diboron
DCC: N,N-dicyclohexylcarbodiiomide
DCM: dichloromethane
DIPEA: N,N-diisopropylethylamine
DMAP: 4-dimethylaminopyridine
DMF dimethylformamide
DMT: dimethoxytrityl
DMTCI: 4,4'dimethoxytrityl chloride
ESI: electrospray ionization
Et: ethyl
EtNH₂: ethyl amine
EtOH: ethanol
FT-IR-ATR: Fourier transform infrared attenuated total reflectance spectroscopy
HATU: 1-[Bis(dimethylamino)methylene]-1H-1,2,3-triazolo[4,5-b]pyridinium 3-oxide hexafluorophosphate
HRMS: high resolution mass spectrometry
JohnPhos: 2-Biphenylyl-di-*tert*.-butylphosphin
KOAc: potassium acetate
Me: methyl
MeCN: acetonitril
MeOH: methanol
NEt₃: triethylamine
Pd(dba)₂: Bis(dibenzylidenaceton)palladium
Pd(dppf)Cl₂: (1,1'-Bis(diphenylphosphino)ferrocene)palladium(II) dichloride
TBAF: tetrabutylammonium fluoride
THF: tetrahydrofuran
TIPSCl: triispropylsilyl chloride
TBDMSO: (1,1-dimethylethyl)dimethylsilyl
SPOS: solid-phase organic synthesis

## Claims

1. A method of synthesizing of 6C modified guanosine analogues, wherein
a) the guanosine analogue comprises 2'deoxy-ribose or ribose,
b) the guanosine analogue is protected at the 5' position by DMT group (dimethoxytrityl),
c) the guanosine analogue is phosphorylated at 3' position with (2-cyanoethoxy)(diisopropylamino)phosphaneyl-group,
d) the guanosine analogue is substituted at the amino group of 2C position with 2-methyl-1-oxopropyl group, and
e) the 6C modification of the guanosine analogue is selected from the group of
a. a heterocycle (C1);
b. a 5-membered heterocycle comprising W, X, Y and Z, wherein W, X, Y and Z can be C, N, O and S (D1);
c. an oxadiazole (D2);
d. a BOC-imidazole (B1) and
e. BOC-pyrazole (B2);
wherein the method comprises the following steps:
i) providing a guanosine precursor molecule, wherein the guanosine precursor is protected by a first and a second protection group at the 3' and 5' positions, wherein the first and the second protection group are selected from:
a. no protection group (A),
b. a TBDMSO group ((1,1-dimethylethyl)dimethylsilyl) (C) and
c. a DMT group (dimethoxytrityl) (D);
and wherein the guanosine precursor molecule is protected by a third protection group at the 6C position, wherein the third protection group is selected from:
a. no protection group (A),
b. 2,4,6-trismethylbenzenesulfonate group (C)
c. and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
a. if the modification group is a heterocycle (C1) or BOC-pyrazole (B2) transfer is performed under conditions: presence of Pd(dba)₂, JohnPhos, K₃PO₄, 1,4-dioxane under reflux, or
b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and BOC protection to form BOC-imidazole (B1),
iv) applying appropriate conditions for adding or removing protection groups to transform the guanosine precursor into 6C modified guanosine analogue, wherein step iv) may be performed at any time during synthesis of 6C modified guanosine analogue and may be performed more than once.

2. The method according to claim 1, wherein at least one step is performed under an inert atmosphere, preferably the inert atmosphere is selected from the group consisting of argon and nitrogen.

3. The method according to claim 1, wherein at least one step performed in a dry (water-free) organic solvent, preferably the solvent is selected from the group consisting of DCM (dichloromethane), THF (tetrahydrofuran), dioxane, DMF (dimethylformamide), MeOH (methanol), MeCN (acetonitrile) and EtOH (ethanol).

4. The method according to claim 1, wherein the precursor molecule, the final product or the intermediate products are protected at least at the 3', 5' positions or the 6C position by suitable protection groups, the protection groups are selected form the group consisting of TBDMSO group ((1,1-dimethylethyl)dimethylsilyl), a DMT group (dimethoxytrityl), a 2,4,6-trismethylbenzenesulfonate group, a BOC group (tert-butyloxycarbonyl), a 2,4,6-tris(1-methylethyl)benzenesulfonate group, isobutyryl group and a PAC group (phenoxyacetyl).

5. A 6C modified guanosine analogue, wherein
a) the guanosine analogue comprises 2'deoxy-ribose or ribose,
b) the guanosine analogue is protected at the 5' position by DMT group (dimethoxytrityl),
c) the guanosine analogue is phosphorylated at 3' position with (2-cyanoethoxy)(diisopropylamino)phosphaneyl-group,
d) the guanosine analogue is substituted at the amino group of 2C position with 2-methyl-1-oxopropyl group, and
e) the 6C modification of the guanosine analogue is selected from the group of a heterocycle (C1); a 5-membered heterocycle comprising W, X, Y and Z, wherein W, X, Y and Z can be C, N, O and S (D1); oxadiazole (D2); BOC-imidazole (B1) and BOC-pyrazole (B2).

6. 6C modified guanosine analogue produced by the following method, wherein the method comprises the following steps:
i) providing a guanosine precursor molecule, wherein the guanosine precursor is protected by a first and a second protection group at the 3' and 5' positions, wherein the first and the second protection group are selected from no protection group (A), TBDMSO group ((1,1-dimethylethyl)dimethylsilyl) (C) and DMT group (dimethoxytrityl) (D); and wherein the guanosine precursor molecule is protected by a third protection group at the 6C position, wherein the third protection group is selected from no protection group (A), 2,4,6-trismethylbenzenesulfonate group (C) and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
a. if the modification group is a heterocycle (C1) or BOC-pyrazole (B2) transfer is performed under conditions: presence of Pd(dba)₂, JohnPhos, K₃PO₄, 1,4-dioxane under reflux, or
b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU, DIPEA, DMF, wherein 4,5-dihydro-imidazole (B1) is processed further by steps comprising oxidation of the imidazoline and BOC protection to form BOC-imidazole (B1),
iv) applying appropriate conditions for adding or removing protection groups to transform the guanosine precursor into 6C modified guanosine analogue, wherein step iv) may be performed at any time during synthesis of 6C modified guanosine analogue and may be performed more than once.

7. A method of synthesizing of 6C modified purine nucleoside analogues, wherein
a) the purine nucleoside analogue comprises 2'deoxy-ribose or ribose,
b) the purine nucleoside analogue is protected at the 5' position by DMT group (dimethoxytrityl),
c) the purine nucleoside analogue is phosphorylated at 3' position with (2-cyanoethoxy)(diisopropylamino)phosphaneyl-group,
d) the 6C modification of the purine nucleoside analogue is selected from the group of
a. a heterocycle (C1);
b. a 5-membered heterocycle comprising W, X, Y and Z, wherein W, X, Y and Z can be C, N, O and S (D1);
c. an oxadiazole (D2);
d. a BOC-imidazole (B1) and
e. BOC-pyrazole (B2);
wherein the method comprises the following steps:
i) providing a purine nucleoside precursor molecule, wherein the purine nucleoside precursor is protected by a first and a second protection group at the 3' and 5' positions, wherein the first and the second protection group are selected from:
a. no protection group (A),
b. a TBDMSO group ((1,1-dimethylethyl)dimethylsilyl) (C) and
c. a DMT group (dimethoxytrityl) (D);
and wherein the purine nucleoside precursor molecule is protected by a third protection group at the 6C position, wherein the third protection group is selected from:
a. no protection group (A),
b. 2,4,6-trismethylbenzenesulfonate group (C)
c. and 2,4,6-tris(1-methylethyl)benzenesulfonate group (D),
ii) providing a second precursor molecule comprising a modification group, wherein the modification group is selected from a heterocycle (C1); a 5-membered heterocycle comprising W, X, Y and Z, wherein W, X, Y and Z can be C, N, O and S (D1); oxadiazole (D2); 4,5-dihydro-imidazole (B1) and BOC-pyrazole (B2),
iii) transferring the modification group from the second precursor molecule to the 6C position of the guanosine precursor molecule, wherein the transfer is performed under conditions selected from the group:
a. if the modification group is a heterocycle (C1) or BOC-pyrazole (B2) transfer is performed under conditions: presence of Pd(dba)₂ ((Bis(dibenzylidenaceton)palladium)), JohnPhos (2-Biphenylyl)-di-tert.-butylphosphin), K₃PO₄, 1,4-dioxane under reflux, or
b. if the modification group is a 5-membered heterocycle comprising W, X, Y and Z, wherein W, X, Y and Z can be C, N, O and S (D1), oxadiazole (D2) or 4,5-dihydro-imidazole (B1) transfer is performed under conditions: presence of HATU (1-[Bis(dimethylamino)methylene]-1H-1,2,3-triazolo[4,5-b]pyridinium 3-oxide hexafluorophosphate), DIPEA (1-[Bis(dimethylamino)methylene]-1H-1,2,3-triazolo[4,5-b]pyridinium 3-oxide hexafluorophosphate), DMF (dimethylformamide), wherein 4,5-dihydro-imidazole (B1) is processed further by steps comprising oxidation of the imidazoline and BOC protection to form BOC-imidazole (B1),
iv) applying appropriate conditions for adding or removing protection groups to transform the purine nucleoside precursor into 6C modified purine nucleoside analogue, wherein step iv) may be performed at any time during synthesis of 6C modified purine nucleoside analogue and may be performed more than once.

8. The method according to claim 7, wherein at least one step is performed under an inert atmosphere, preferably the inert atmosphere is selected from the group consisting of argon and nitrogen.

9. The method according to claim 7, wherein at least one step performed in a dry (water-free) organic solvent, preferably the solvent is selected from the group consisting of DCM (dichloromethane), THF (tetrahydrofuran), dioxane, DMF (dimethylformamide), MeOH (methanol), MeCN (acetonitrile) and EtOH (ethanol).

10. The method according to claim 7, wherein the precursor molecule, the final product or the intermediate products are protected at least at the 3', 5' positions or the 6C position by suitable protection groups, the protection groups are selected form the group consisting of TBDMSO group ((1,1-dimethylethyl)dimethylsilyl), a DMT group (dimethoxytrityl), a 2,4,6-trismethylbenzenesulfonate group, a BOC group (tert-butyloxycarbonyl), a 2,4,6-tris(1-methylethyl)benzenesulfonate group, isobutyryl group and a PAC group (phenoxyacetyl).

11. The method according to claim 7, wherein the purine is selected from the group consisting of adenine, guanine, hypoxanthine, xanthine, theophylline, theobromine, caffeine, uric acid, isoguanine and inosine.

12. A 6C modified purine nucleoside analogue, wherein
a) the purine nucleoside analogue comprises 2'deoxy-ribose or ribose,
b) the purine nucleoside analogue is protected at the 5' position by DMT group (dimethoxytrityl),
c) the purine nucleoside analogue is phosphorylated at 3' position with (2-cyanoethoxy)(diisopropylamino)phosphaneyl-group,
d) the 6C modification of the purine nucleoside analogue is selected from the group consisting of
a. a heterocycle (C1);
b. a 5-membered heterocycle comprising W, X, Y and Z, wherein W, X, Y and Z can be C, N, O and S (D1);
c. an oxadiazole (D2);
d. a BOC-imidazole (B1);
e. BOC-pyrazole (B2); and
f. an amide substituted with H, Me, Et, EtNH₂, propargyl or other side chains, especially containing amines or alkynes (E1).
